(19) Europäisches Patentamt
European Patent Office
Office européen des brevets

(11) Numéro de publication : **0 655 238 A1**

# (12) DEMANDE DE BREVET EUROPEEN

(21) Numéro de dépôt : **94402731.7**

(51) Int. Cl.$^6$ : **A61K 9/127**

(22) Date de dépôt : **29.11.94**

(30) Priorité : **30.11.93 FR 9314336**

(43) Date de publication de la demande :
**31.05.95 Bulletin 95/22**

(84) Etats contractants désignés :
**AT BE CH DE DK ES FR GB GR IE IT LI LU MC NL PT SE**

(71) Demandeur : **CENTRE NATIONAL DE LA RECHERCHE SCIENTIFIQUE (CNRS)**
**3, rue Michel Ange**
**F-75016 Paris (FR)**

(72) Inventeur : **Ollivon, Michel**
**20, rue Mot**
**F-94120 Fontenay Sous Bois (FR)**
Inventeur : **Lesieur, Sylviane, 12 Boulevard Desgranges**
**10 Résidence,**
**"La Pépinière"**
**F-92330 Sceaux (FR)**
Inventeur : **Chopineau, Joel**
**7, Impasse Jeannetot**
**F-60200 Compiegne (FR)**
Inventeur : **Puisieux, Francis**
**66, rue de Strasbourg**
**F-94700 Maisons Alfort (FR)**
Inventeur : **Thomas, Daniel**
**Domaine de Rimberlieu,**
**33 allée de l'Etang**
**F-60150 Villers Sur Coudin (FR)**

(74) Mandataire : **Le Guen, Gérard et al**
**CABINET LAVOIX**
**2, place d'Estienne d'Orves**
**F-75441 Paris Cédex 09 (FR)**

## (54) Procédé de préparation de liposomes par voie enzymatique.

(57) La présente invention a pour objet un procédé de préparation de liposomes qui consiste à :
— mettre en contact un ou plusieurs lipide(s) et un ou plusieurs agents tensio-actifs en milieu aqueux, de manière à former des agrégats mixtes formés du(des) lipide(s) et du(des) agent(s) tensio-actif(s), au moins un des agents tensio-actifs étant un substrat enzymatique ;
— éliminer partiellement ou totalement du(des) agent(s) tensio-actif(s) au moyen d'une ou plusieurs enzyme(s), pour former des vésicules lipidiques.

EP 0 655 238 A1

L'invention a pour objet un procédé de préparation de liposomes par voie enzymatique.

Les liposomes sont des vésicules de taille microscopique ou submicroscopique qui existent naturellement dans certaines cellules. Leur membrane lipidique de quelques nanomètres d'épaisseur sépare deux milieux aqueux, l'un interne l'autre externe, qui peuvent être de compositions différentes. Ils peuvent être considérés comme des unités élémentaires de volume, utilisées soit comme réservoir soit comme unité de transport. Ces propriétés les rendent particulièrement appropriés à un grand nombre d'applications, notamment pharmaceutiques ou cosmétiques. Les liposomes utilisés dans ces domaines sont obtenus de manière synthétique sur des échelles qui vont du milligramme à la tonne.

Les liposomes qui sont généralement fabriqués dans l'eau ou des solutés aqueux, sont principalement obtenus à l'échelle industrielle ou du laboratoire par des techniques mécaniques, thermiques ou chimiques, la nature seule fabricant des liposomes par des voies biologiques, lesquelles sont encore mal connues. Les liposomes obtenus artificiellement sont uni-, oligo- ou multilamellaires selon la technique employée et les conditions de fabrication choisies.

A ce jour, il n'a pas été décrit dans la littérature un procédé permettant d'obtenir de manière reproductible à l'échelle industrielle des liposomes par voie enzymatique.

F.F. CHAO et al. (J. Biol. Chem., 267 (1992), 4992-4998) ont décrit la possibilité de reproduire in vitro des liposomes fabriqués in vivo à partir de la lipoprotéine plasmatique de faible densité humaine (LDL) en hydrolysant les esters de cholestérol de la LDL à l'aide d'une cholestérol estérase après prétraitement de la LDL à l'aide de trypsine. Cette expérimentation, mettant en jeu un assemblage complexe de lipoprotéines et un ester de cholestérol de type particulier ne donne aucune indication à l'homme du métier sur la possibilité de produire des liposomes à l'échelle industrielle à partir de lipides seuls par voie enzymatique, dans la mesure où l'ester de cholestérol formant le noyau de la LDL n'est pas apte à "solubiliser" les lipides et à former des aggrégats mixtes avec les lipides susceptibles de former des vésicules, ceci en l'absence de protéine et en milieu aqueux. Dans toutes les techniques utilisant des agents de surface, cette formation d'agrégats mixtes constitue un préalable essentiel à la préparation des liposomes.

Les inventeurs auteurs de la présente invention ont maintenant découvert qu'il était possible d'obtenir dans des conditions douces, de manière reproductible à l'échelle industrielle, des liposomes par voie enzymatique.

L'invention a pour objet un procédé de préparation de liposomes comprenant :

- la mise en contact d'un ou plusieurs lipide(s) et d'un ou plusieurs agent(s) tensio-actifs ou assimilés en milieu aqueux, de manière à former des agrégats mixtes formés du(des) lipide(s) et du(des) agent(s) tensio-actif(s), au moins un des agents tensio-actifs étant un substrat enzymatique ;
- l'élimination partielle ou totale du(des) agent(s) tensio-actif(s) des agrégats mixtes au moyen d'une ou plusieurs réaction(s) enzymatique(s) pour former des vésicules lipidiques.

La première étape du procédé est commune à toutes les techniques de préparation de liposomes dites par élimination de tensio-actif et ayant pour point de départ la formation de systèmes colloïdaux mixtes dans lesquels les lipides sont incorporés, si ce n'est que les agents promouvant la formation de ces colloïdes avec les lipides mis en oeuvre sont des agents tensio-actifs qui remplissent un certain nombre de conditions qui sont les suivantes :

- ces agents sont des substrats pour une ou plusieurs enzymes,
- leur élimination, obtenue par hydrolyse de la molécule initiale ou transformation de celle-ci, doit conduire à des produits ne perturbant pas l'existence des vésicules, l'obtention de ces dernières étant conditionnée par la possibilité d'organisation des lipides en phase lamellaire, qu'ils y soient ou non associés à des produits de la réaction enzymatique. Ces produits doivent donc être compatibles avec cette organisation, soit parce qu'ils ne la modifient pas ou peu, les lipides s'organisant déjà de cette manière en leur absence, soit parce qu'ils la favorisent en étant absolument nécessaires, les lipides seuls ne s'organisant pas de cette manière. En particulier, la quantité de substance doit être adaptée pour obtenir la formation d'aggrégats ouverts, c'est-à-dire ne contenant pas de compartiment interne aqueux, à partir des lipides et doit être suffisamment faible pour que la quantité de produit résultant de la (des) réaction(s) enzymatique(s) ne déstabilise pas les phases lamellaires fermées ;
- les produits résultants peuvent être :
  - a) - entièrement solubles dans l'eau,
  - b) - entièrement solubles dans les constituants de la membrane des liposomes,
  - c) - totalement insolubles dans l'un ou les deux milieux,
  - d) - partiellement solubles dans l'un ou les deux milieux précédents,
  - e) - ou toute combinaison de ces cas de figures,

et ne doivent pas présenter de caractéristiques incompatibles avec l'utilisation proposée des liposomes. Et même dans certains cas, les produits obtenus seront des agents stabilisants des liposomes ou permettant de leur donner des caractéristiques particulières, par exemple le cholestérol sera utilisé comme produit de la réaction pour rendre la bicouche des liposomes imperméable aux solutés aqueux. La formation des liposomes en tant qu'agrégat lamellaire fermé et capable de retenir des substances étrangères est induite par la ou les réaction(s) enzymatique(s) selon un schéma général de réaction :

Enzyme(s) + agent(s) tensio-actif(s) → produits choisis parmi a) à e).

Les produits a) à e), par exemple le(s) lipide(s) compatible(s) et le(s) produit(s) soluble(s) sont tels que définis ci-dessus.

Les agents tensio-actifs selon l'invention sont des substances amphiphiles répondant aux critères énoncés ci-dessus possédant une partie hydrophobe et une partie hydrophile,

- la partie hydrophobe étant avantageusement constituée d'une ou plusieurs chaînes hydrocarbonées, de préférence de $C_6$ à $C_{18}$, saturées ou insaturées, linéaires ou ramifiées, cycliques ou acycliques, aromatiques ou non aromatiques,
- la partie hydrophile étant ionique ou nonionique et avantageusement constituée d'un reste glucidique, d'un reste polyol, d'un reste alcool, d'un reste amine, d'un groupe polyoxyéthylène et/ou polyoxypropylène, d'un reste acide carboxylique, sulfurique, sulfonique, phosphonique ou phosphorique, ou d'une association de ces restes.

L'agent tensio-actif est de préférence choisi parmi les dérivés d'alcool gras ou d'acides gras, de préférence en $C_6$-$C_{18}$ et de sucre, notamment les alkylglucosides et les sucroesters, ou parmi les dérivés amphiphiles des stérols obtenus par l'association à un reste stérol d'une partie hydrophile monomère ou polymère, notamment les (poly)osides ou dérivés polyoxyéthylés du cholestérol.

Parmi les dérivés d'alcool gras et de sucre, on peut citer notamment les dérivés du n-dodécanol et de sucre, tel que le dodécylmaltoside.

Parmi les dérivés de stérols, on peut citer notamment les dérivés du cholestérol, tels que les osides du cholestérol, ou les esters du cholestérol tels que le sébaçate de cholestérol polyéthoxylé.

Les lipides de départ sont choisis parmi tout lipide ou mélange de lipides utilisé pour la fabrication des liposomes, notamment les glycérides, par exemple les phospholipides tels que les dérivés de phosphatidylcholine, notamment la dipalmitoyl phosphatidylcholine ou des dérivés polyglycérolés à longues chaînes tels que l'hexadécyl éther de diglycérol.

L'enzyme est choisie en fonction de la structure de l'agent tensio-actif. On citera notamment à titre d'enzymes appropriées les glucosidases, les amyloglucosidases, les estérases et les phosphatases.

De manière avantageuse, on peut utiliser des systèmes à enzymes immobilisées.

La nature et la quantité d'agent tensio-actif sont choisies en fonction du lipide constituant les phases lamellaires, de façon à ce que les produits de dégradation soient compatibles avec l'existence de phases lamellaires comme il est connu de la littérature. Ainsi, A.G. LEE (Biochemistry 15 (1976) p. 2448-2454) a démontré que les phases lamellaires des phosphatidylcholines pures pouvaient s'accommoder de la présence d'alcools gras (jusqu'à 66% en mole) quand la longueur des chaînes saturées de ces derniers étaient plus courtes de deux à quatre carbones que celle des phospholipides (par exemple une membrane de dipalmitoyl phosphatidylcholine-(DPPC) pourra accepter jusqu'à 66% de n-dodécanol tout en restant en phase lamellaire). Il est donc possible de retenir le dodécanol comme produit final d'une réaction enzymatique visant à fabriquer des vésicules de DPPC. Comme pour toutes les réactions dont le produit final résulte d'une coupure de l'agent tensio-actif, la partie la plus hydrophobe (ici le dodécanol) se retrouvera quasiment en totalité dans la bicouche étant donné sa faible solubilité dans l'eau.

Dans le cas où l'on utilise la DPPC comme lipide, le dodécylmaltoside (DM) comme agent tensio-actif et une amyloglucosidase ou une α-glucosidase et une β-D-glucosidase qui dégradent l'agent tensio-actif suivant les réactions :

$$DM + H_2O \xrightarrow{\text{amyloglucosidase/ } \alpha\text{-glucosidase}} \text{Dodécylglucoside (DG)} + \text{glucose}$$

$$DG + H_2O \xrightarrow{\beta\text{-D-glucosidase}} \text{Dodécanol} + \text{glucose}$$

le rapport molaire agent tensio-actif / lipide est de 1,5 ou moins.

Lorsque le rapport molaire agent tensio-actif/lipide est supérieur à 1,5, la limite de saturation des phases lamellaires par l'agent tensio-actif sera atteinte ou dépassée.

La mise en contact de molécules de phospholipides et de dodécylmaltoside donne des micelles mixtes. L'élimination de l'agent tensio-actif par hydrolyse enzymatique conduit à la formation de vésicules de taille submicronique et variable avec la vitesse de la réaction d'hydrolyse. L'encapsulation d'un principe actif peut se faire en même temps que l'élimination de l'agent tensio-actif par voie enzymatique.

Dans le cas où l'on utilise des dérivés du cholestérol, dont l'élimination enzymatique conduira à la formation de cholestérol libre, ceux-ci seront ajoutés dans les proportions qui permettent d'obtenir les propriétés souhaitées de la bicouche des liposomes, par exemple : imperméabilisation d'une bicouche de phospholipides par ajout de 30 à 50% en poids de cholestérol, ou stabilisation d'une bicouche d'hexadécyl éther de diglycérol par ajout de 50% en poids de cholestérol, par rapport au poids des lipides totaux.

Lorsque l'on utilise un phospholipide comme lipide de départ, l'agent tensio-actif est avantageusement un oside de cholestérol ou un ester de cholestéryle tel que le sébaçate de cholestérol polyéthoxylé. Dans ce cas, les vésicules finales contiendront du cholestérol après hydrolyse de l'agent tensio-actif à l'aide d'une osidase ou d'une estérase suivant le cas.

La première étape du procédé se déroule dans des conditions classiques bien connues de l'homme du métier et comprend la préparation d'une solution contenant le(les) lipide(s) de départ et le(les) agent(s) tensio-actif(s). Eventuellement, on ajoute à la solution, outre l'agent tensio-actif constituant un substrat enzymatique, toute substance ou mélange de substances compatible(s) avec la fermeture des vésicules et l'existence de phase lamellaire en fin de réaction qui pourrait apporter des propriétés spécifiques aux liposomes par exemple, un(des) lipide(s) chargé(s) dont la présence empêche l'agrégation des vésicules, un second tensio-actif, une(des) molécule(s)se fixant en surface des liposomes et permettant leur reconnaissance ultérieure, un(des) marqueur(s) spécifique(s), une(des) protéine(s), une(des) enzyme(s) ou toute(s) séquence(s) d'acides aminés ou d'acides nucléiques pourvu qu'ils n'empêchent pas le processus enzymatique de formation des liposomes. Ces composés peuvent également être ajoutés à toute autre étape du processus de formation des vésicules. La préparation a lieu à une température compatible avec la stabilité des produits mis en oeuvre et généralement comprise entre 20 et 60°C, de préférence à une températuer entre 20 et 40°C.

Lorsque l'on obtient un des produits a), c), d) ou e) précédemment mentionnés, le(s) produit(s) non soluble(s) dans les constituants de la membrane des liposomes, peuvent être éliminés par toute technique appropriée (cristallisation, filtration, chromatographie, etc...).

La seconde étape du procédé se déroule en milieu aqueux tamponné dans une gamme de température compatible avec les transitions micelles → vésicules, de préférence une température comprise entre 20 et 60°C, ou encore mieux 20 et 40°C. Durant la réaction enzymatique, la masse du système reste constante.

L'(les) enzyme(s) peu(ven)t être introduite(s), soit sous forme lyophilisée à laquelle la solution contenant les colloïdes destinés à être transformés en vésicules est ajoutée, soit être fixées sur un support pour obtention immédiate de liposomes par passage sur colonne par exemple. L'ajustement des paramètres gouvernant la(les) cinétique(s) enzymatique(s) (quantité d'enzyme, pH, température, agitation, etc. ) permet de moduler la vitesse d'obtention des liposomes et également leur taille moyenne.

La réaction se déroule pendant une durée pouvant atteindre plusieurs heures, sous agitation douce. La cinétique enzymatique peut être suivie au travers de l'évolution de la taille des agrégats par mesure de la turbidité, une augmentation brutale de la turbidité suivie par un passage par un maximum étant caractéristique de la formation des vésicules (M. OLLIVON et al., Biochemistry 27-5 (1988) p. 1685-1703).

La possibilité d'encapsuler un principe actif où tout composé intéressant peut avoir lieu en même temps que se forment les liposomes dans des conditions douces. L'élimination de l'enzyme à la fin du processus n'est pas forcément nécessaire ; l'ajout d'un inhibiteur compétitif dans le milieu à la fin du procédé ou d'un agent chimique spécifique du site actif peut être envisagée pour stopper la réaction à un stade prédéterminé.

La disparition totale ou partielle de l'agent tensio-actif peut donner naissance ou laisser dans la membrane des éléments stabilisateurs de ces dernières. Ces éléments sont soit liposolubles (stérols), soit amphiphiles (le groupement hydrosoluble pouvant être (poly)osidique, polyoxyéthylé) ; certains de ces composés étant parfois ajoutés intentionnellement aux préparations liposomales pour les stabiliser ou obtenir des propriétés particulières (ex. : imperméabilisation des membranes par le cholestérol), comme décrit par G. VANLERBERGHE et al., Colloque National du CNRS, n° 938 (Physico-chimie des composés amphiphiles, Ed. R. PERRON (1979), p. 303-311). Quels que soient les lipides et les agents tensio-actifs utilisés, l'invention permet à partir de la "solubilisation" partielle de ces lipides par un ou plusieurs de ces agents tensio-actifs, sous forme de micelles ou d'agrégats mixtes, d'obtenir des liposomes pouvant inclure un ou plusieurs principes actifs, que ceux-ci soient hydro ou liposolubles ou amphipathiques. Le procédé se prête à la fabrication extemporanée de liposomes, puisqu'il suffit pour préparer des liposomes de mélanger l'enzyme aux lipides présolubilisés,

comme dans les exemples donnés ci-après.

Les avantages du procédé sont qu'aucun moyen mécanique ni aucun solvant ne sont nécessaires. Le procédé est réalisé dans des conditions dosées en milieu aqueux à des températures modérées compatibles avec l'activité des enzymes choisies. En outre, le procédé selon l'invention permet la fabrication extemporanée de liposomes, et donc l'encapsulation de substances labiles.

**EXEMPLE 1**

Fabrication de vésicules non-ioniques contenant du cholestérol :

1 mg d'estérase (enzyme obtenue sous forme lyophilisée avec une activité de 500 U.I./mg) est ajoutée sous forme d'une solution 1 (50 µl) contenant environ 20 mg/ml d'enzyme à 1.15 ml de solution 2 contenant :

2.45 µM d'hexadécyl éther de diglycérol ($C_{16}G_2$)

0.2 µM de dicétylphosphate (DCP)

3.0 µM de sébacate de cholestérol polyéthoxylé (Chol POE).

La réaction a été suivie à 39,5° C sous légère agitation pendant 15 heures par mesure de la turbidité à 400 nm (spectophotomètre UV-VIS, lambda 2, Perkin-Elmer). L'augmentation brutale de la turbidité suivie par un passage par un maximum est caractéristique de la formation de vésicules. La réaction se poursuit pendant plusieurs heures. La suspension turbide obtenue est analysée par diffusion quasi-élastique de la lumière et montre la formation d'agrégats d'environ 0.1 µm de diamètre hydrodynamique moyen.

L'encapsulation d'une solution d'un marqueur fluorescent hydrosoluble (calcéine) à la concentration de 1 mM a été pratiquée parallèlement et de manière identique à la précédente. L'élution des objets formés dans cette solution sur une colonne de chromatographie d'exclusion sur gel (Séphacryl S1000) couplée à un spectrofluorimètre (SPEX) capable d'une double détection simultanée à 509 et 516 nm (excitation à 489 nm) a permis d'une part de confirmer la taille moyenne de ces objets (0.1 µm) et d'autre part de montrer qu'il s'agissait de vésicules (encapsulation du marqueur).

La microscopie électronique par transmission pratiquée sur ces échantillons marqués par coloration négative a également permis de confirmer l'existence d'objets fermés d'apparence identique à celle de vésicules témoins contenant les mêmes lipides et préparés par une méthode classique utilisant l'ultrasonication.

Il a été montré par des analyses similaires que le dérivé triglycérolé (hexadécyl éther de triglycérol statistique ($C_{16}$-$G_3$) présentait le même comportement et était donc lui aussi capable de donner naissance dans les mêmes conditions à des vésicules par voie enzymatique.

**EXEMPLE 2**

L'exemple 2 illustre la formation de liposomes de phospholipides obtenus par la double réaction enzymatique :

dodécylmaltoside ($C_{12}M$) + amyloglucosidase $\rightarrow$ dodécylglucose ($C_{12}G$)

$C_{12}G$ + β glucosidase $\rightarrow$ alcool dodécylique ($C_{12}OH$)

qui a lieu en présence de dipalmitoyl phosphatidylcholine (DPPC) en milieu aqueux tamponé et qui peut être résumée par :

$$\text{micelles mixtes de } (DPPC+C_{12}M) + \text{solution tampon} \xrightarrow{\text{enzyme(s)}} \text{vésicules de } (C_{12}OH+DPPC) + \text{sucres dans solution tampon.}$$

A 20 ml d'une solution tamponée (NaCl=145mM, HEPES=10mM, pH 7,4) de micelles mixtes de DPPC dans le dodécylmaltoside contenant :

10 µM de dipalmitoyl phosphatidylcholine (DPPC) et

15 µM de dodécylmaltoside ($C_{12}M$), sont ajoutés à 37°C

200 µl d'une solution enzymatique composée de 1.15 mg d'amyloglucosidase (57 U.I.) et de 3.l mg de β glucosidase (62 U.I.) dans le tampon.

Les variations de la turbidité qui sont enregistrées à 400 nm en continu (spectrophotomètre UV-VIS lambda 2, Perkin-Elmer) pendant 5 heures en milieu agité et non agité sur une partie aliquote (2 ml) montrent, après une brève période de latence, de très importants accroissements de turbidité. Ces augmentations, qui sont suivies d'une décroissance ou d'un quasi-palier de turbidité, correspondent à la formation de vésicules (M. OLLIVON et al., Biochemistry, 27-5, (1988), p. 1685-1703). Au fur et à mesure de la consommation du $C_{12}M$ par les réactions enzymatiques et de sa transformation en $C_{12}G$ puis $C_{12}OH$, les micelles mixtes initialement présentes dans la solution de départ sont progressivement transformées en vésicules. Les réactions enzymatiques permettent de décrire le chemin inverse de celui de la solubilisation enregistré par ailleurs en présence et en l'absence de dodécanol. Les parties riches en eau (tampon NaCl, HEPES, ci-dessus) des diagrammes de phase DPPC-$C_{12}M$-eau et DPPC+$C_{12}OH$(1:2 mol-mol)-$C_{12}M$-eau ont été déterminées en l'absence d'enzyme à partir de l'exploitation des courbes de variation de turbidité enregistrées lors de l'ajout en continu de solutions de $C_{12}M$ à des suspensions de liposomes de concentrations en lipides variables (LESIEUR et al., Chem. Phys. of Lipids, 56-2 (1990) p. 109-122). Ces diagrammes montrent d'une part que la limite de solubilité micellaire (limite du diagramme de phase telle que pour ces concentrations de $C_{12}M$ et au-delà uniquement des micelles mixtes sont observées) en l'absence de dodécanol peut être assimilée à une droite dont les coefficients sont définis par la relation :

$$[C_{12}M] = 1.5 \times [DPPC] + 0.07,$$

$[C_{12}M]$ et [DPPC] étant respectivement les concentrations de $C_{12}M$ et de DPPC présentes, et d'autre part que ces limites sont très sensiblement augmentées en présence de dodécanol, cette même limite étant déterminée par la relation :

$$[C_{12}M] = 2.6 \times [DPPC] + 0.4,$$

cependant que la limite inférieure d'existence de ces micelles (limite à partir de laquelle les vésicules sont progressivement transformées en micelles mixtes), définie par la relation :

$$[C_{12}M] = 1.43 \times [DPPC] + 0.04$$

est sensiblement inférieure aux deux précédentes. La formation de dodécanol dans la bicouche entraînera une accélération du processus de la transition micelle-vésicule par rapport au même processus enregistré en l'absence de cet alcool.

La taille moyenne des agrégats formés mesurés par diffusion quasi-élastique de la lumière est comprise entre 0.2 et 0.3 μm.

L'encapsulation d'une solution d'un marqueur fluorescent hydrosoluble (calcéine) à la concentration de 1 mM a été pratiquée parallèlement et de manière identique à la précédente. L'élution des objets formés dans cette solution sur une colonne de chromatographie d'exclusion sur gel (Séphacryl S1000) couplée à un spectrofluorimètre (SPEX) capable d'une double détection simultanée à 509 et 516 nm (excitation à 489 nm) a permis d'une part de confirmer la taille moyenne de ces objets (0.1 μm) et d'autre part de montrer qu'il s'agissait de vésicules (encapsulation du marqueur).

## Revendications

1. Procédé de préparation de liposomes comprenant :
   - la mise en contact d'un ou plusieurs lipide(s) et d'un ou plusieurs agents tensio-actifs en milieu aqueux, de manière à former des agrégats mixtes formés du(des) lipide(s) et du(des) agent(s) tensio-actif(s), au moins un des agents tensio-actifs étant un substrat enzymatique ;
   - l'élimination partielle ou totale du(des) agent(s) tensio-actif(s) au moyen d'une ou plusieurs enzyme(s), pour former des vésicules lipidiques.

2. Procédé selon la revendication 1, caractérisé en ce que l'agent tensio-actif constituant un substrat enzymatique est un substrat pour plusieurs enzymes différentes.

3. Procédé selon la revendication 1, caractérisé en ce que l'agent tensio-actif constituant un substrat enzymatique comprend une partie hydrophobe constituée d'une ou plusieurs chaînes hydrocarbonées saturées ou insaturées, linéaires ou ramifiées, cycliques ou acycliques, aromatiques ou non aromatiques.

4. Procédé selon la revendication 1 ou la revendication 2, caractérisé en ce que l'agent tensio-actif constituant le substrat enzymatique comprend une partie hydrophobe constituée d'un noyau stérolique.

5. Procédé selon l'une quelconque des revendications précédentes, caractérisé en ce que l'agent tensio-actif constituant le substrat enzymatique comprend une partie hydrophile ionique ou non ionique choisie parmi les restes de glucides, de polyols, d'alcools, d'amines, d'oxyde d'éthylène, d'oxyde de propylène,

d'acide carboxylique, d'acide sulfurique, sulfonique, phosphonique ou phosphorique, ou plusieurs de ces restes.

**6.** Procédé selon l'une des revendications précédentes, caractérisé en ce que l'agent tensio-actif constituant un substrat enzymatique est choisi parmi les dérivés d'alcool gras ou d'acides gras, ou parmi les dérivés amphiphiles des stérols obtenus par l'association à un reste stérol d'une partie hydrophile monomère ou polymère, notamment les (poly)osides ou dérivés polyoxyéthylés du cholestérol.

**7.** Procédé selon l'une des revendications précédentes, caractérisé en ce que l'agent tensio-actif constituant un substrat enzymatique est choisi parmi les dérivés d'alcool gras ou d'acides gras, et d'un sucre.

**8.** Procédé selon la revendication 1, caractérisé en ce que l'enzyme est choisi parmi une glucosidase, une amyloglucosidase, une estérase et une phosphatase.

**9.** Procédé selon la revendication 1, caractérisé en ce que l'on prépare des liposomes à partir de dipalmitoylphosphatidylcholine et de dodécylmaltoside, au moyen d'une amyloglucosidase ou une $\alpha$-glucosidase et d'une $\beta$-glucosidase.

**10.** Procédé selon la revendication 1, caractérisé en ce que l'on prépare des liposomes à partir d'hexadécyl éther de di- ou de triglycérol et de sébaçate de cholestérol polyéthoxylé, éventuellement additionné d'un autre agent tensio-actif ionique, notamment le dodécylphosphate au moyen d'une estérase.

**11.** Procédé selon la revendication 10, caractérisé en ce que le sébaçate de cholestérol polyéthoxylé est ajouté à l'hexadécyl éther de di- ou triglycérol à raison de 30 à 50% par rapport à l'hexadécyl éther de di- ou triglycérol.

**12.** Procédé selon l'une des revendications précédentes, caractérisé en ce que lors de l'élimination de l'agent tensio-actif, on ajoute au milieu réactionnel un principe actif de façon à réaliser l'encapsulation de celui-ci.

Office européen des brevets

# RAPPORT DE RECHERCHE EUROPEENNE

Numero de la demande
EP 94 40 2731

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | WO-A-85 01440 (CENTRE NATIONAL DE LA RECHERCHE SCIENTIFIQUE (C.N.R.S.) ET AL.)<br>* le document en entier *<br>--- | 1-12 | A61K9/127 |
| D,A | THE JOURNAL OF BIOLOGICAL CHEMISTRY, vol.267, no.7, 5 Mars 1992, BALTIMORE (US) pages 4992 - 4998<br>FEI-FEI CHAO ET AL. 'hydrolysis of cholesteryl ester in low density lipoprotein converts this lipoprotein to a liposome'<br>* le document en entier *<br>----- | 1-12 | |

DOMAINES TECHNIQUES RECHERCHES (Int.Cl.6)
A61K

Le présent rapport a été établi pour toutes les revendications

| Lieu de la recherche | Date d'achèvement de la recherche | Examinateur |
|---|---|---|
| LA HAYE | 6 Mars 1995 | Benz, K |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)